# EUROPEAN PATENT APPLICATION

(11) **EP 3 721 722 A1**
(43) Date of publication of application: **14.10.2020**
(21) Application number: 19169001.5
(22) Date of filing: 12.04.2019
(51) Int. Cl.: A24B 15/16, A24F 47/00, A61K 31/465

(54) **A METHOD OF MANUFACTURING**

(71) Applicant: NERUDIA LIMITED, Liverpool Merseyside L24 9HP (GB)
(72) Inventor: The designation of the inventor has not yet been filed
(74) Representative: Mewburn Ellis LLP

(57) **Abstract**

A method for manufacturing a nicotine-dosed freeze-dried plant material includes the steps of combining a nicotinic compound with a plant material, and freeze-drying the plant material.

## Description

### Field of the Invention

The present invention relates to a method of manufacturing a nicotine-dosed freeze-dried plant material, and a nicotine-dosed freeze-dried plant material product.

### Background

Smoking substitute devices, which may also be known as electronic nicotine delivery systems, may comprise electronic systems that permit a user to simulate the act of smoking by producing an aerosol, also referred to as a "vapour", which is drawn into the lungs through the mouth (inhaled) and then exhaled. The inhaled aerosol typically bears nicotine and/or flavourings without, or with fewer of, the odour and health risks associated with traditional smoking.

In general, smoking substitute devices are intended to provide a substitute for the rituals of smoking, whilst providing the user with a similar experience and satisfaction to those experienced with traditional smoking and tobacco products.

The popularity and use of smoking substitute devices has grown rapidly in the past few years. Although originally marketed as an aid to assist habitual smokers wishing to quit tobacco smoking, consumers are increasingly viewing smoking substitute devices as desirable lifestyle accessories. Some smoking substitute devices are designed to resemble a traditional cigarette and are cylindrical in form with a mouthpiece at one end. Other smoking substitute devices do not generally resemble a cigarette (for example, the smoking substitute device may have a generally box-like form).

There are a number of different categories of smoking substitute devices, each utilising a different smoking substitute approach.

One approach for a smoking substitute device is the so-called "vaping" approach, in which a vaporisable liquid, typically referred to as "e-liquid", is heated by a heating device to produce an aerosol vapour which is inhaled by a user. An e-liquid typically includes a base liquid as well as nicotine and/or flavourings. The resulting vapour therefore typically contains nicotine and/or flavourings.

A typical vaping smoking substitute device includes a mouthpiece, a power source (typically a battery), a tank for containing e-liquid, as well as a heating device. In use, electrical energy is supplied from the power source to the heating device, which heats the e-liquid to produce an aerosol (or "vapour") which is inhaled by a user through the mouthpiece.

Vaping smoking substitute devices can be configured in a variety of ways. For example, there are "closed system" vaping smoking substitute devices which typically have a sealed tank and heating element which is pre-filled with e-liquid and is not intended to be refilled by an end user. One subset of closed system vaping smoking substitute devices include a main body which includes the power source, wherein the main body is configured to be physically and electrically coupled to a consumable including the tank and the heating element. In this way, when the tank of a consumable has been emptied, the main body can be reused by connecting it to a new consumable. Another subset of closed system vaping smoking substitute devices are completely disposable, and intended for one-use only.

There are also "open system" vaping smoking substitute devices which typically have a tank that is configured to be refilled by a user, so the device can be used multiple times.

An example vaping smoking substitute device is the myblu™ e-cigarette. The myblu™ e cigarette is a closed system device which includes a main body and a consumable. The main body and consumable are physically and electrically coupled together by pushing the consumable into the main body. The main body includes a rechargeable battery. The consumable includes a mouthpiece, a sealed tank which contains e-liquid, as well as a heating device, which for this device is a heating filament coiled around a portion of a wick which is partially immersed in the e-liquid. The device is activated when a microprocessor on board the main body detects a user inhaling through the mouthpiece. When the device is activated, electrical energy is supplied from the power source to the heating device, which heats e-liquid from the tank to produce a vapour which is inhaled by a user through the mouthpiece.

Another example vaping smoking substitute device is the blu PRO™ e-cigarette. The blu PRO™ e cigarette is an open system device which includes a main body, a (refillable) tank, and a mouthpiece. The main body and tank are physically and electrically coupled together by screwing one to the other. The mouthpiece and refillable tank are physically coupled together by screwing one into the other, and detaching the mouthpiece from the refillable tank allows the tank to be refilled with e-liquid. The device is activated by a button on the main body. When the device is activated, electrical energy is supplied from the power source to a heating device, which heats e-liquid from the tank to produce a vapour which is inhaled by a user through the mouthpiece.

Another approach for a smoking substitute device is the so-called "heat not burn" ("HNB") approach in which tobacco (rather than e-liquid) is heated or warmed to release vapour. The tobacco may be leaf tobacco or reconstituted tobacco. The vapour may contain nicotine and/or flavourings. In the HNB approach the intention is that the tobacco is heated but not burned, i.e. does not undergo combustion.

A typical HNB smoking substitute device may include a main body and a consumable. The consumable may include the tobacco material. The main body and consumable may be configured to be physically coupled together. In use, heat may be imparted to the tobacco material by a heating device that is typically located in the main body, wherein airflow through the tobacco material causes moisture in the tobacco material to be released as vapour. A vapour may be formed from a carrier in the tobacco material and additionally volatile compounds released from the tobacco. The released vapour may be entrained in the airflow drawn through the tobacco.

As the vapour passes through the smoking substitute device (entrained in the airflow) from an inlet to a mouthpiece (outlet), the vapour cools and condenses to form an aerosol (also referred to as a vapour) for inhalation by the user. The aerosol will normally contain the volatile compounds.

An example of the HNB approach is the IQOS® smoking substitute device from Philip Morris Ltd. The IQOS® smoking substitute device uses a consumable, including reconstituted tobacco located in a wrapper. The consumable includes a holder incorporating a mouthpiece. The consumable may be inserted into a main body that includes a heating device. The heating device has a thermally conductive heating knife which penetrates the reconstituted tobacco of the consumable, when the consumable is inserted into the heating device. Activation of the heating device heats the heating element (in this case a heating knife), which, in turn, heats the tobacco in the consumable. The heating of the tobacco causes it to release nicotine vapour and flavourings which may be drawn through the mouthpiece by the user through inhalation.

A second example of the HNB approach is the device known as "Glo"® from British American Tobacco p.l.c. Glo® comprises a relatively thin consumable. The consumable includes leaf tobacco which is heated by a heating device located in a main body. When the consumable is placed in the main body, the tobacco is surrounded by a heating element of the heating device. Activation of the heating device heats the heating element, which, in turn, heats the tobacco in the consumable. The heating of the tobacco causes it to release nicotine vapour and flavourings which may be drawn through the consumable by the user through inhalation. The tobacco, when heated by the heating device, is configured to produce vapour when heated rather than when burned (as in a smoking apparatus, e.g. a cigarette). The tobacco may contain high levels of aerosol formers (carrier).

A host of e-liquid formulations are commercially available for use in vaping smoking substitute devices.

Generally e-liquids do not contain solid material as this can interfere with the capillary action of the wick and cause problems with clogging. There have been prior proposals for enabling a consumer to infuse base liquid with solid material in order to customise the flavour of the e-liquid. However, the intention is that solid material is removed prior to supplying the e-liquid to a device for vaporization.

Although vaping smoking substitute devices are gaining popularity in many parts of the world, they are not universally acceptable for use and in a significant number of countries the sale or supply of nicotine-containing e-liquids is restricted. This may be due to the fact that in many countries purified nicotine is classified as a pharmaceutical product and for this reason the sale and use of nicotine-containing liquids is highly regulated. Such countries may in fact prohibit the sale of nicotine-containing e-liquids.

In some countries vaping smoking devices are considered to be drug delivery devices and consequently their use and / or sale may also be regulated or prohibited.

It would be desirable to provide a vaping smoking substitute device and system which can be used to deliver an aerosol containing a nicotine component and which would be acceptable for sale and use in countries which currently do not permit the sale and use of purified nicotine-containing e-liquids due to their classification as drugs or pharmaceutical products.

In particular, it would also be highly desirable to provide a vaping smoking substitute system which does not require the consumer to purchase a purified nicotine-containing liquid. It would also be desirable to provide a vaping smoking substitute system which does not require the consumer to handle a purified nicotine-containing liquid.

### Summary of the Invention

At its most general, the present invention relates to a method for manufacturing a freeze-dried plant material.

According to a first aspect of the present invention, there is provided a method of manufacturing a nicotine-dosed freeze-dried plant material comprising:
combining a nicotinic compound with a plant material; and
freeze-drying the plant material.

As used herein, the term "nicotinic compound" or "nicotinic" is intended to refer to nicotine, nicotine salt(s), nicotine complex(es), and/or nicotine solvate(s).

As used herein, the term "nicotine-dosed" is intended refer to a material that has been dosed with a "nicotinic compound" (i.e. the dosed content is not inherent to the material itself).

As used herein, the term "plant material" is intended to refer to a portion and/or part(s) of a plant (e.g. leaf, stem, flower or bud). The plant material may be processed (for example, by shredding, grinding or drying) or it may be non-processed (that is, used whole). The plant material is typically fibrous (comprising or characterised by fibres). For the avoidance of doubt the term "plant material" is not intended to include pulp and/or paper which is derived from a plant material and chemically and/or mechanically processed to extract fibres before use.

In this way, the nicotinic content of the plant material can be controlled so as to provide a more consistent and improved sensory experience to the user. In particular, the nicotinic content may be increased. Also, freeze-drying of the plant material provides a product with a longer shelf life. This is beneficial for storage and transportation of the product.

Preferably, the method comprises combining the nicotine-dosed freeze-dried plant material with an aerosol-former liquid to provide a suspension of the nicotine-dosed freeze-dried plant material in the aerosol-former liquid.

In this way, delivery of nicotinic compound to the aerosol-former liquid is achieved by infusion of the nicotinic compound from the nicotine-dosed freeze-dried plant material. The aerosol-former liquid may be referred to as an e-liquid. This allows controlled delivery of the nicotinic compound to the aerosol-former liquid in a short period of time. Also, other active compounds inherent to the plant material may infuse into the aerosol-former liquid to provide the user with a sensory experience having the inherent qualities of the plant material (e.g. taste, aroma, and/or aesthetic effect) when using the suspension in a vaping smoking substitute device.

Examples of active compounds that may be inherent to the plant material include, but are not limited to, nicotinic compounds (e.g. nicotine), cocaine, caffeine, opiates and opioids, cathine and cathinone, kavalactones, mysticin, beta-carboline alkaloids, salvinorin A together with any combinations, functional equivalents to, and/or synthetic alternatives of the foregoing.

Preferably, the nicotinic compound is combined with the plant material before freeze-drying the plant material.

In this way, improved impregnation and uniform distribution of the nicotinic compound with the plant material is achieved. This results in a more efficient and reproducible delivery of nicotinic compound to the plant material.

Preferably, the nicotinic compound comprises a nicotine salt selected from nicotine hydrochloride, nicotine dihydrochloride, nicotine monotartrate, nicotine bitartrate, nicotine bitartrate dihydrate, nicotine sulphate, nicotine zinc chloride monohydrate and nicotine salicylate, and combinations thereof.

In this way, these salts provide the user with a satisfactory nicotine "hit" and additionally provide an improved sensory experience. Furthermore, nicotine salts exhibit lower volatility than nicotine and consequently are less likely to be lost during the freeze-drying process.

Preferably, the aerosol-former liquid is a nicotine-free liquid.

In this way, the consumer is not required to handle a purified nicotine-containing liquid. As nicotine is a controlled substance this mitigates the risk associated with a user handling such purified nicotine-containing liquids.

Optionally, the aerosol-former liquid may also comprise water.

Conveniently, the nicotine-dosed freeze-dried plant material is visible in the aerosol-former liquid such that this identifies the nicotinic compound as being sourced from the plant material. Preferably the nicotine-dosed freeze-dried plant material is visible in the consumable for a vaping smoking substitute device. Preferably the nicotine-dosed freeze-dried plant material is visible in the vaping smoking substitute device.

Preferably, the aerosol-former liquid is a polyhydric alcohol.

Advantageously, good dispersion of the nicotinic compound is achieved in the aerosol-former liquid when the aerosol-former liquid is a polyhydric alcohol.

Suitable polyhydric alcohols include propylene glycol (PG), triethylene glycol, 1,2-butane diol and vegetable glycerine (VG)) and their esters (e.g. glycerol mono-, di- or tri-acetate). It is most preferred that the polyol is propylene glycol or vegetable glycerine or combinations thereof.

Advantageously, propylene glycol or vegetable glycerine exhibit minimal toxicity. Furthermore, they impart a sweet taste that is satisfactory to the user.

In preferred embodiments the suspension comprises propylene glycol and vegetable glycerine in a ratio within the range of 10:90 to 90:10 by volume, preferably 20:80 to 80:20, more preferably 25:75 to 75: 25, more preferably 30:70 to 70:30, and most preferably 40:60 to 60:40 by volume.

In preferred embodiments the suspension comprises propylene glycol and vegetable glycerine in a ratio within the range of 10:90 to 90:10 by weight, preferably 20:80 to 80:20, more preferably 25:75 to 75: 25, more preferably 30:70 to 70:30, and most preferably 40:60 to 60:40 by weight.

Optionally, the aerosol-former liquid may include optional additives such as flavourants. It is preferred the flavourants are in liquid form.

Any suitable plant material that may be used within a vaping smoking substitute device may be used in the present invention.

The plant material may comprise least one plant material selected from the list including Amaranthus dubius, Arctostaphylos uva-ursi (Bearberry), Argemone mexicana, Amica, Artemisia vulgaris, Yellow Tees, Galea zacatechichi, Canavalia maritima (Baybean), Cecropia mexicana (Guamura), Cestrum noctumum, Cynoglossum virginianum (wild comfrey), Cytisus scoparius, Damiana, Entada rheedii, Eschscholzia califomica (California Poppy), Fittonia albivenis, Hippobroma longiflora, Humulus japonica (Japanese Hops), Humulus lupulus (Hops), Lactuca virosa (Lettuce Opium), Laggera alata, Leonotis leonurus, Leonurus cardiaca (Motherwort), Leonurus sibiricus (Honeyweed), Lobelia cardinalis, Lobelia inflata (Indian-tobacco), Lobelia siphilitica, Nepeta cataria (Catnip), Nicotiana species (Tobacco), Nymphaea alba (White Lily), Nymphaea caerulea (Blue Lily), Opium poppy, Passiflora incamata (Passionflower), Pedicularis densiflora (Indian Warrior), Pedicularis groenlandica (Elephant's Head), Salvia divinorum, Salvia dorrii (Tobacco Sage), Salvia species (Sage), Scutellaria galericulata, Scutellaria lateriflora, Scutellaria nana, Scutellaria species (Skullcap), Sida acuta (Wireweed), Sida rhombifolia, Silene capensis, Syzygium aromaticum (Clove), Tagetes lucida (Mexican Tarragon), Tarchonanthus camphoratus, Tumera diffusa (Damiana), Verbascum (Mullein), Zamia latifolia (Maconha Brava) together with any combinations, functional equivalents to, and/or synthetic alternatives of the foregoing.

Preferably, the plant material is tobacco.

In this way, as the invention is primarily intended to for use as, or part of, a vaping smoking substitute consumable, the use of tobacco provides a consumable having the inherent properties of tobacco, for example the texture, aroma and flavour.

Optionally, the tobacco is in the form of tobacco leaf and/or tobacco stem.

The method according to the first aspect of the invention may further comprise a step of making a smoking substitute consumable. In particular, a smoking substitute consumable for vaping. Preferably, the smoking substitute consumable is a vaping smoking substitute consumable.

According to a second aspect of the present invention, there is provided a nicotine-dosed freeze-dried plant material product produced by a process according to the first aspect of the invention, wherein the amount of nicotinic compound added to the plant material is between 0.1 to 25 weight % of the total weight of the nicotine-dosed freeze-dried plant material.

In this way, a plant material product for a vaping smoking substitute device with increased amounts of nicotinic compound is provided. This allows the user to experience a stronger nicotine "hit". In addition, a plant material product with an enriched nicotinic content and longer shelf-life is provided. Also, where the nicotine-dosed freeze-dried plant material product is added to an aerosol-former liquid it enables smaller doses of nicotine-dosed freeze-dried plant material product to be added without reducing the amount of nicotine delivered.

The amount of nicotinic compound added to the plant material may have a value of at least 0.1 wt % of the total weight of the nicotine-dosed freeze-dried plant material product, such as at least 0.5 wt %, such as at least 1 wt %, such as at least 2 wt%, such as at least 5 wt %, such as at least 10 wt%, such as at least 15 wt %, such as at least 20 wt %, such as at least 25 wt%.

The amount of nicotinic compound added to the plant material may have a value of at most 0.1 wt % of the total weight of the freeze-dried plant material product, such as at most 0.5 wt %, such as at most 1 wt %, such as at most 2 wt%, such as at most 5 wt %, such as at most 10 wt%, such as at most 15 wt %, such as at most 20 wt %, such as at most 25 wt%.

Preferably, the amount of nicotinic compound added to the plant material is between 5 to 25 weight % of the total weight of the nicotine-dosed freeze-dried plant material, such as 10 to 25 weight %, such as 15 to 25 weight %.

In this way, a plant material product for a vaping smoking substitute device with a higher nicotinic content is achieved. This enables the nicotine to be tailored to suit the user's requirements.

Preferably, the nicotinic compound comprises a nicotine salt selected from nicotine hydrochloride, nicotine dihydrochloride, nicotine monotartrate, nicotine bitartrate, nicotine bitartrate dihydrate, nicotine sulphate, nicotine zinc chloride monohydrate and nicotine salicylate, and combinations thereof.

In this way, these salts provide the user with a satisfactory nicotine "hit". They also provide an improved sensory experience to the user. Additionally, they show good solubility characteristics for many aerosol-former liquids.

Preferably, the nicotine-dosed freeze-dried plant material product comprises a suspension of the nicotine-dosed freeze-dried plant material product in an aerosol-former liquid, wherein the suspension contains 0.1 to 20 weight % of the nicotine-dosed freeze-dried plant material product based on the total weight of the suspension.

The suspension may contain at least 0.1 wt % of nicotine-dosed freeze-dried plant material product based on the total weight of the suspension, such as at least 0.5 wt %, such as at least 1 wt %, such as at least 2 wt %, such as at least 5 wt %, such as at least 10 wt %, such as at least 15 wt %, such as at least 20 wt %.

The suspension may contain at most 0.1 wt % of nicotine-dosed freeze-dried plant material product based on the total weight of the suspension, such as at most 0.5 wt %, such as at most 1 wt %, such as at most 2 wt %, such as at most 5 wt %, such as at most 10 wt %, such as at most 15 wt %, such as at most 20 wt %.

In this way, the suspension is provided with qualities inherent to the plant material (e.g. flavour and aroma) to provide an improved sensory experience to the user. Additionally, an enhanced amount of nicotinic compound is delivered to the aerosol-former liquid.

Often the suspension may contain at least 1% by weight, preferably at least 2% by weight, more preferably at least 3% and especially at least 4% by weight of the nicotine-dosed freeze-dried plant material product. In some embodiments the suspension may contain up to 19% or 18% by weight of nicotine-dosed freeze-dried plant material product, preferably up to 17 % or 16% and more preferably up to 15% by weight of nicotine-dosed freeze-dried plant material product.

Preferably, the suspension comprises at least 2.0 mg/ml of nicotinic compound.

In this way, the user is supplied with a sufficient dose of nicotinic compound per ml of aerosol-former liquid.

Some embodiments may contain at least 2.5 mg / ml, preferably at least 2.8 mg / ml of nicotinic compound, more preferably at least 3.0 mg, most preferably at least 3.2 mg and especially at least 3.5 mg of nicotinic compound per ml of total liquid. The amount of nicotinic compound present can be determined by HPLC methods as known in this technical field.

Typically the maximum amount of nicotinic compound in the aerosol-former liquid would be 7.0 mg / ml, preferably 6.5 mg / ml, more preferably 6.0 mg / ml, and further preferably 5.5 mg / ml.

Preferably, the nicotine-dosed freeze-dried plant material product is particulate, wherein the particle size is between 20 µm to 2000 µm.

Very small particles of nicotine-dosed freeze-dried plant material are best avoided as they can become entrained with the vapour and be inhaled by the user and this is regarded as unpleasant. Large particle sizes are also more likely to cause problems with clogging of the wick of the vaping smoking substitute device. Nicotine-dosed freeze-dried plant material solids having particle sizes in a range of 50 to 2000µm, particularly 100 to 1000µm and especially 250 to 500 µm have provided an excellent combination of efficient release of nicotinic compound together with resistance to clogging of the wick of the consumable plant material.

In this way, an efficient ratio of surface area to liquid is provided to achieve an effective release of nicotinic compound. It is preferred that very small nicotinic-dosed freeze-dried plant material solids are avoided as they are more likely to cause problems with clogging of the vaping smoking substitute device, or even be small enough to be released with the vapour when the consumable is in use.

According to a third aspect of the present invention, there is provided use of the nicotine-dosed freeze-dried plant material product according to the second aspect of the invention in a vaping smoking substitute device.

According to a fourth aspect of the present invention, there is provided a kit comprising a nicotine-dosed freeze-dried plant material product produced by the process according to the first aspect of the invention, or the nicotine-dosed freeze-dried plant material product according to the second aspect of the invention, and an aerosol-former liquid.

### Summary of the Figures

So that the invention may be understood, and so that further aspects and features thereof may be appreciated, embodiments illustrating the principles of the invention will now be discussed in further detail with reference to the accompanying figures, in which:

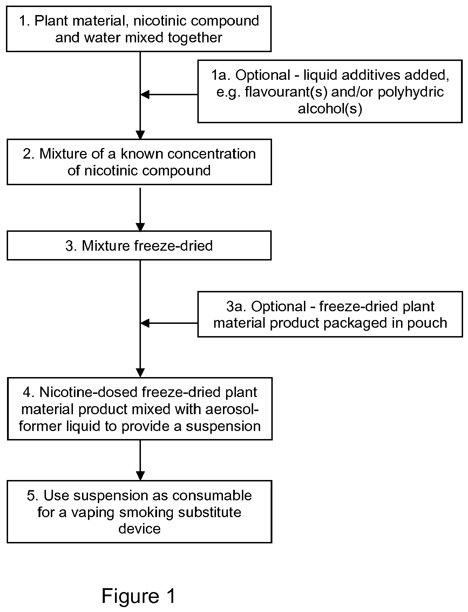

**Figure 1****.** is an illustrative view of a manufacturing process for a nicotine-dosed freeze-dried plant material product for use in accordance with the present invention.

### Detailed Description of the Invention

Aspects and embodiments of the present invention will now be discussed with reference to the accompanying figures. Further aspects and embodiments will be apparent to those skilled in the art. All documents mentioned in this text are incorporated herein by reference.

Figure 1 illustrates a preferred embodiment of the invention. With reference to Figure 1 this embodiment is described in detail below.

Initially, the plant material is combined with water and a nicotinic compound 1 to provide a mixture of known concentration of nicotinic compound **2.** The amount of nicotinic compound combined may be tailored to the content required in the final product.

Optional additives such as flavourants and/or polyhydric alcohols may be added to the mixture, **1a.** It is preferred that the additives are liquid. Suitable flavourants include Mild Menthol, Mixed Berry, Cool Menthol, Peach, Cream Cake, Melon Medley, Apple, Shisha, Grape, Virginia Tobacco, and American Red Blend. Suitable polyhydric alcohols include propylene glycol (PG), triethylene glycol, 1,2-butane diol and vegetable glycerine (VG)) and their esters (e.g. glycerol mono-, di- or tri-acetate). It is most preferred that the polyhydric alcohol is propylene glycol or vegetable glycerine or combinations thereof.

The mixture is then freeze-dried **3** using a freeze-drying machine. Typical freeze-drying machines are manifold freeze-dryers, shelf freeze-dryers and rotary freeze-dryers.

Freeze-drying, also known as lyophilisation or cryodessication, uses low temperatures to remove water/moisture from a material. The process involves freezing the material, then lowering the pressure to facilitate removal of ice from the material by sublimation. The main stages of freeze-drying are freezing and annealing, primary drying and secondary drying.

In the freezing stage the mixture is cooled below its triple point to ensure sublimation, rather than melting, occurs in the subsequent stages. It is preferred that large crystals are formed during the freezing stage to provide faster and more efficient freeze-drying. To produce large crystals the mixture should be cooled slowly, or annealed by cycling the temperature up and down. Typical freezing temperatures are between -50 °C and -80°C.

In the primary drying phase the pressure is lowered through the application of a partial vacuum, and heat is provided to facilitate sublimation of the ice. In this phase about 95 % of the water is sublimed. Organic solvents are also reduced and/or removed during primary drying.

The secondary drying phase is intended to remove remaining unfrozen water molecules that are bound to the product. The temperature in this phase is higher than that in the primary phase to break any physicochemical interactions formed between the water molecules and the frozen material. Temperatures can be above 0 °C. The water content of the product after this stage is typically < 5 %.

Optionally, after freeze-drying the nicotine-dosed freeze-dried plant material product may be packaged in a pouch **3a.** Such that when the pouch is immersed within an aerosol-former liquid it facilitates infusion of soluble components contained within the nicotine-dosed freeze-dried plant material into the aerosol-former liquid, whilst retaining the insoluble freeze-dried plant material components within the pouch.

The nicotine-dosed freeze-dried plant material product is then mixed with an aerosol-former liquid, such as propylene glycol and/or vegetable glycerine, to provide a suspension comprising insoluble plant material within the aerosol-former liquid **4.** Soluble components of the nicotine-dosed freeze-dried plant material product such as nicotinic compound and optional flavourants dissolve within the aerosol-former liquid to form a nicotinic compound (and optionally flavour) enriched aerosol-former liquid.

The nicotine-dosed freeze-dried plant material product of the present invention may be used as a consumable, or part of a consumable for a vaping smoking substitute device **5.**

The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the invention in diverse forms thereof.

While the invention has been described in conjunction with the exemplary embodiments described above, many equivalent modifications and variations will be apparent to those skilled in the art when given this disclosure. Accordingly, the exemplary embodiments of the invention set forth above are considered to be illustrative and not limiting. Various changes to the described embodiments may be made without departing from the spirit and scope of the invention.

For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

Throughout this specification, including the claims which follow, unless the context requires otherwise, the words "have", "comprise", and "include", and variations such as "having", "comprises", "comprising", and "including" will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means, for example, +/- 10%.

The words "preferred" and "preferably" are used herein refer to embodiments of the invention that may provide certain benefits under some circumstances. It is to be appreciated, however, that other embodiments may also be preferred under the same or different circumstances. The recitation of one or more preferred embodiments therefore does not mean or imply that other embodiments are not useful, and is not intended to exclude other embodiments from the scope of the disclosure, or from the scope of the claims.

## Claims

1. A method of manufacturing a nicotine-dosed freeze-dried plant material comprising:
combining a nicotinic compound with a plant material; and
freeze-drying the plant material.

2. The method according to claim 1 comprising:
combining the nicotine-dosed freeze-dried plant material with an aerosol-former liquid to provide a suspension of the nicotine-dosed freeze-dried plant material in the aerosol-former liquid.

3. The method according to any preceding claim, wherein the nicotinic compound is combined with the plant material before freeze-drying the plant material.

4. The method according to any preceding claim, wherein the nicotinic compound comprises a nicotine salt selected from nicotine hydrochloride, nicotine dihydrochloride, nicotine monotartrate, nicotine bitartrate, nicotine bitartrate dihydrate, nicotine sulphate, nicotine zinc chloride monohydrate and nicotine salicylate, and combinations thereof.

5. The method according to any preceding claim, wherein the aerosol-former liquid is a nicotine-free liquid.

6. The method according to any preceding claim, wherein the aerosol-former liquid is a polyhydric alcohol.

7. The method according to any preceding claim, wherein the plant material is tobacco.

8. A nicotine-dosed freeze-dried plant material product produced by a process according to any preceding claim, wherein the amount of nicotinic compound added to the plant material is between 0.1 to 25 weight % of the total weight of the nicotine-dosed freeze-dried plant material.

9. The nicotine-dosed freeze-dried plant material product according to claim 8, wherein the nicotinic compound comprises a nicotine salt selected from nicotine hydrochloride, nicotine dihydrochloride, nicotine monotartrate, nicotine bitartrate, nicotine bitartrate dihydrate, nicotine sulphate, nicotine zinc chloride monohydrate and nicotine salicylate, and combinations thereof.

10. The nicotine-dosed freeze-dried plant material product according to claim 8 or claim 9, comprising:
a suspension of the nicotine-dosed freeze-dried plant material product in an aerosol-former liquid, wherein the suspension contains 0.1 to 20 weight % of the nicotine-dosed freeze-dried plant material product based on the total weight of the suspension.

11. The nicotine-dosed freeze-dried plant material product according to claim 10, wherein the suspension comprises at least 2.0 mg/ml of the nicotinic compound.

12. The nicotine-dosed freeze-dried plant material product according to any one of claims 8 to 11,
wherein the nicotine-dosed freeze-dried plant material product is particulate, and wherein the particle size is between 20 µm to 2000 µm.

13. Use of the nicotine-dosed freeze-dried plant material product according to any one of claims 8 to 12 in a vaping smoking substitute device.

14. A kit comprising a nicotine-dosed freeze-dried plant material product produced by the process of claims 1 to 7, or the nicotine-dosed freeze-dried plant material product according to claims 8 to 12, and an aerosol-former liquid.
